# DEMANDE DE BREVET EUROPEEN

(11) **EP 1 308 142 A2**
(43) Date de publication de la demande: **07.05.2003**
(21) Numéro de dépôt: 02356214.3
(22) Date de dépôt: 29.10.2002
(51) Int. Cl.: A61F 2/38

(54) **Implant rotulien et prothèse de genou incorporant un tel implant**

(30) Priorité: 30.10.2001 FR 0114068
(71) Demandeur: TORNIER SA, 38330 Saint-Ismier (FR)
(72) Inventeur: Tornier, Alain, 38330 Saint Ismier (FR)
(74) Mandataire: Myon, Gérard

(57) **Abrégé**

Cet implant rotulien pour articulation prothétique totale ou partielle de genou incorpore une surface articulaire externe (31) et une surface articulaire interne (32) prévues pour coopérer respectivement avec une berge externe et une berge interne d'une trochlée fémorale ou d'un composant prothétique fémoral et reliées entre elles par une arête de transition (33). L'arête (33) est courbe en vue de face de l'implant (3), avec une concavité tournée vers la surface articulaire externe (31), alors que la surface articulaire externe (31) est concave dans un plan parallèle au plan sagittal et dans un plan transversal.

## Description

L'invention a trait à un implant rotulien, et à une prothèse totale ou partielle de genou comprenant un tel implant.

Dans le domaine des prothèses de genou, il est connu d'équiper une rotule naturelle d'un implant destiné à former une ou des surfaces articulaires prévues pour coopérer avec les surfaces articulaires fémorales, qu'il s'agisse de surfaces naturelles ou, le plus souvent, de surfaces prothétiques.

Il est connu, par exemple de US-A-5,609,640 ou de EP-A-0 021 421, de réaliser un implant rotulien en forme de dôme sphérique ou de chapeau de gendarme. Compte tenu de sa géométrie, un tel implant est en contact ponctuel ou quasi ponctuel avec chaque condyle, ce qui induit des contraintes localisées importantes dans l'implant d'une part et dans le composant prothétique fémoral ou le fémur d'autre part. Il est également connu de FR-A-2 700 260 de réaliser une pièce rotulienne avec deux surfaces articulaires, de forme sphérique et concave, qui sont symétriques par rapport au plan sagittal et séparées par une surface de liaison également symétrique par rapport à ce plan. Compte tenu de la géométrie de la surface de liaison, les surfaces articulaires ne peuvent généralement pas être en appui surfacique contre la trochlée pendant tout un mouvement de flexion ou d'extension du genou.

Cette congruence imparfaite entre les surfaces articulaires prévues respectivement sur l'implant rotulien et sur le fémur résulte également dans une instabilité médio-latérale de la rotule, ce qui peut conduire à un positionnement non naturel de la rotule. Ce défaut de congruence induit également une instabilité de la rotule en pivotement dans le plan sagittal, ce qui diminue l'efficacité du muscle extenseur et tend à augmenter l'usure des surfaces articulaires en contact. Compte tenu de ces limitations, la pose d'un tel implant doit être effectuée de façon très précise, faute de quoi il ne peut pas remplir sa fonction.

C'est à ces inconvénients qu'entend plus particulièrement remédier l'invention en proposant un implant rotulien dont la géométrie est adaptée pour permettre un appui efficace sur la trochlée du fémur, cet appui demeurant congruent dans les différentes positions de l'articulation du genou

Dans cet esprit, l'invention concerne un implant du type précité, qui comprend une surface articulaire externe et une surface articulaire interne prévues pour coopérer respectivement avec une berge externe et une berge interne d'une trochlée fémorale ou d'un composant prothétique fémoral et reliées entre elles par une arête de transition, alors que la surface articulaire externe est concave dans un plan parallèle au plan sagittal de l'articulation et dans un plan transversal. Cet implant est caractérisé en ce que l'arête de transition est courbe en vue de face de l'implant, avec une concavité tournée vers la surface articulaire externe.

Au sens de la présente invention, l'adjectif « interne » correspond au côté de l'articulation d'un genou orienté vers l'autre genou, alors que l'adjectif « externe » correspond au côté orienté à l'opposé de l'autre genou. Le plan sagittal est un plan coupant le genou d'arrière en avant dans une zone médiane, alors qu'un plan transversal est un plan perpendiculaire aux tendons rotuliens et aux tendons du quadriceps.

Grâce à l'invention, la congruence des surfaces articulaires prévues sur l'implant et la trochlée est largement améliorée par rapport à l'état de la technique. En particulier, la concavité de la surface articulaire externe dans les deux plans perpendiculaires l'un par rapport à l'autre permet d'augmenter la stabilité du contact et de diminuer l'usure de cette surface. Cette double concavité, éventuellement associée à la convexité de la surface articulaire interne, permet également d'ajuster la rotule par un pivotement autour de la berge externe du fémur, de telle sorte que l'implant et la rotule sont en permanence correctement positionnés par rapport aux condyles et à la trochlée, sans perte de congruence. Le caractère courbe de l'arête contribue à la définition de la surface articulaire externe et est compatible avec le mouvement d'ajustement précité, cette arête se trouvant engagée dans la trochlée. L'invention prend le parti délibéré de favoriser la congruence des surfaces articulaires externes prévues respectivement sur l'implant et au niveau de la berge externe trochléenne ou du condyle externe, qu'il s'agisse d'un condyle naturel ou d'un élément prothétique, car l'essentiel des efforts d'appui de la rotule sur les condyles transitent par ces surfaces externes.

Selon des aspects avantageux mais non obligatoires de l'invention, l'implant incorpore une ou plusieurs des caractéristiques suivantes :
- Le centre de courbure de l'arête de transition est situé à l'extérieur ou à la périphérie de l'implant, en vue de face. Ceci correspond au fait que le rayon de courbure de cette arête est relativement important.
- La surface articulaire externe de l'implant est une surface de révolution.
- L'implant a, en vue de face, une forme globalement ovoïde.
- La surface articulaire interne de l'implant est plane ou convexe dans un plan parallèle au plan sagittal. Le fait que cette surface soit plane ou convexe dans un plan parallèle au plan sagittal et qu'elle offre moins de congruence est moins favorable que la situation relative à la surface articulaire externe, ce qui n'est pas rédhibitoire car les efforts transitant par cette surface sont moins importants que ceux transitant par la surface articulaire externe. De plus, le caractère plan ou convexe de cette surface dans un tel plan permet le glissement de cette surface sur le condyle interne lors d'un mouvement d'ajustement de l'implant, par pivotement de sa surface articulaire externe sur le condyle externe.
- Deux chanfreins latéraux s'étendent selon des directions globalement perpendiculaires au plan sagittal et relient les bords respectifs des surfaces articulaires à un bord périphérique de l'implant. Ces chanfreins évitent un blocage éventuel de l'implant sur un coin ou un bord d'une surface prothétique fémorale.
- L'implant est réalisé dans une pièce plastique monobloc, ce qui lui permet d'avoir une épaisseur suffisante, sans risque de contact métal/métal entre l'élément prothétique fémoral et un plateau de renforcement, tel qu'il en existe dans un implant bi-matière.

L'invention concerne également une prothèse totale ou partielle du genou qui comprend un implant tel que précédemment décrit. Une telle prothèse est plus facile à implanter pour le chirurgien dans la mesure où le positionnement de l'implant peut être effectué avec une plus grande tolérance, l'implant ayant tendance à ajuster sa position par rapport au condyle de part la congruence de sa surface articulaire externe et du condyle externe. Une telle prothèse a également moins tendance à s'user que les prothèses connues.

L'invention sera mieux comprise et d'autres avantages de celle-ci apparaîtront plus clairement à la lumière de la description qui va suivre de deux modes de réalisation d'un implant rotulien et d'une prothèse conformes à son principe, donnée uniquement à titre d'exemple et faite en référence aux dessins annexés dans lesquels :
- la figure 1 est une vue de face d'une articulation de genou équipée d'une prothèse conforme à l'invention ;
- la figure 2 est une coupe de la rotule selon la ligne II-II à la figure 1 ;
- la figure 3 est une vue de face de l'implant rotulien utilisé dans la prothèse des figures 1 et 2 ;
- la figure 4 est une coupe selon la ligne IV-IV à la figure 3 ;
- la figure 5 est une coupe selon la ligne V-V à la figure 3 ;
- la figure 6 est une vue en perspective de l'implant des figures 3 à 5 vu du côté de sa face avant ;
- la figure 7 est une vue en perspective de l'implant des figures 3 à 6 vu du côté de sa face arrière et
- la figure 8 est une vue analogue à la figure 3 pour un implant conforme à un second mode de réalisation de l'invention.

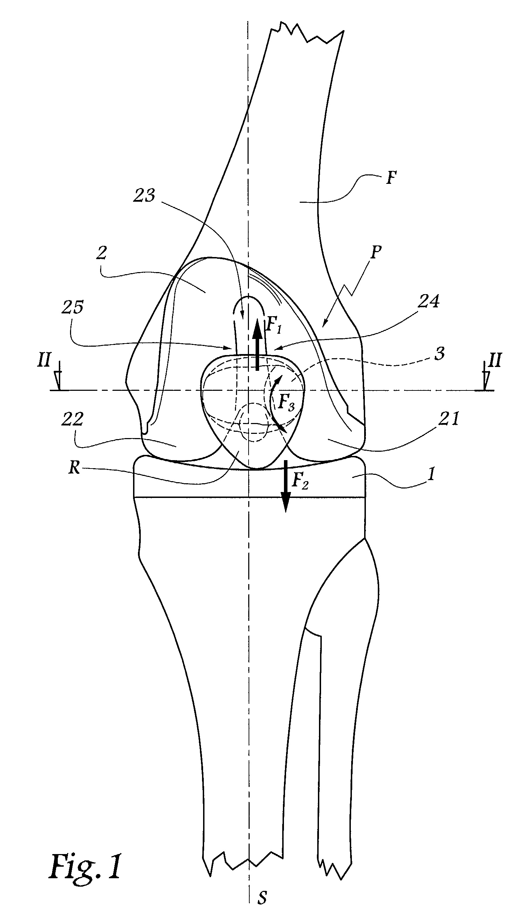

La prothèse P représentée à la figure 1 comprend un composant prothétique tibial 1, un composant prothétique fémoral 2 et un implant rotulien 3 destiné à être implanté dans la rotule R après résection de celle-ci.

Dans l'exemple représenté, le genou en question est un genou gauche, de sorte que son côté interne se situe sur la gauche de la figure 1 alors que son côté externe se situe sur la droite de celle-ci.

Le composants 2 forme deux condyles 21 et 22, respectivement externe et interne, ainsi que deux berges externe 24 et interne 25 de la trochlée fémorale. Ces deux condyles et ces deux berges sont destinés à recevoir en appui l'implant 3.

L'implant 3 est susceptible de mouvements de translation par rapport à la trochlée 23 définie par le composant 2, ces mouvements étant représentés par les flèches F₁ et F₂.

L'implant 3 est également susceptible de mouvements de pivotement autour du condyle 21, comme représenté par la double flèche F₃.

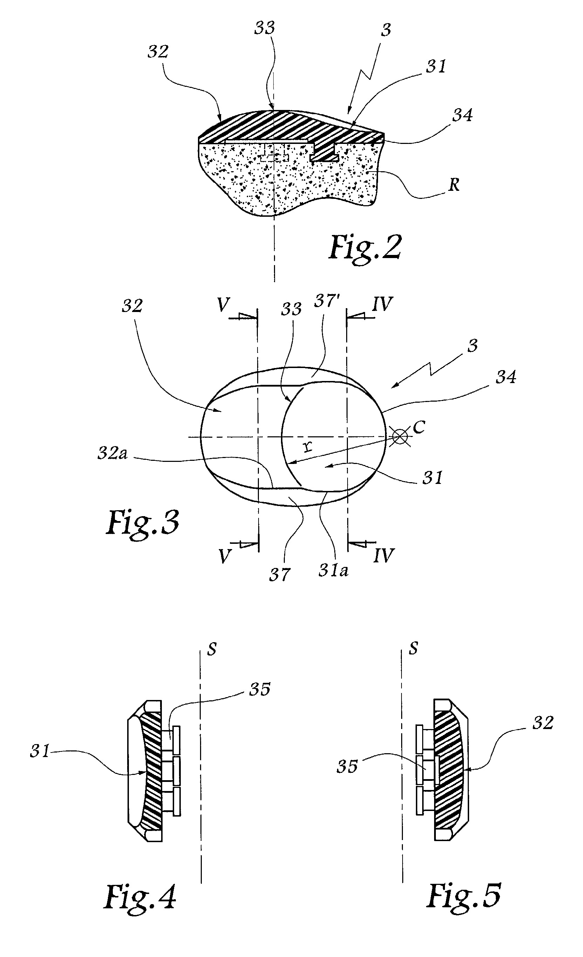

La géométrie de l'implant 3 ressort des figures 2 à 7.

Cet implant comprend une surface articulaire externe 31 destinée à venir en appui contre le condyle 21 ou la berge externe 24 et une surface articulaire interne 32 destinée à venir en appui contre le condyle 22 ou la berge interne 25.

Une arête de transition 33 relie les surfaces 31 et 32. Cette arête n'est pas vive, en ce sens qu'elle est arrondie.

La surface 31 est concave dans le plan de la figure 4, c'est-à-dire dans un plan parallèle au plan sagittal S de l'articulation représenté par sa trace aux figures 1 et 2.

Dans le plan de la figure 2, qui est un plan transversal par rapport à l'articulation, cette surface 31 est également concave.

Ainsi, la surface 31 est bi-concave et peut glisser et pivoter dans le sens de la flèche F₃ sur le condyle 21 qui est bi-convexe.

La surface 32 est, quant à elle, convexe dans le plan de la figure 5, qui est également parallèle au plan sagittal S, et convexe dans le plan de la figure 2.

Selon des variantes de l'invention, la surface 32 pourrait être plane dans le plan de la figure 5, voire concave dans le plan de la figure 2.

Comme il ressort plus particulièrement de la figure 3, l'arête 33 est courbe, avec une concavité tournée vers la surface 31.

On note C le centre de courbure de l'arête 33. Comme il ressort de la figure 3, ce centre de courbure est situé à l'extérieur de l'implant 3, du côté de la surface 31, de sorte que le rayon de courbure r de l'arête 33 est relativement important. En variante, le centre C pourrait être défini au niveau du bord périphérique 34 de l'implant 3.

Du fait de la géométrie des surfaces 31 et 32 et de l'arête 33, on obtient un appui surfacique des surfaces 31 et 21 l'une sur l'autre alors que l'appui des surfaces 32 et 22 l'une sur l'autre est linéique ou quasi-ponctuel. Ceci est justifié par le fait que l'essentiel des efforts de compression de la rotule R en direction du fémur F transite par les surfaces 31 et 21.

La géométrie de l'arête 33 lui permet de s'engager sans difficulté dans la trochlée 33 et d'être compatible avec des déplacements de la rotule R par rapport au fémur F représentés par les flèches F₁ à F₃.

L'implant 3 est également pourvu de trois plots 35 d'encrage dans la rotule R et d'une rainure 36 globalement en forme de Y qui sert à favoriser l'ancrage de l'implant 3 dans un lit de ciment.

En pratique, la surface 31 est conçue de telle sorte qu'elle constitue une surface de révolution, notamment autour d'un axe perpendiculaire au plan de la figure 3.

L'implant 3 est également pourvu de deux chanfreins latéraux 37 et 37' qui relient les bords respectifs 31a et 32a des surfaces 31 et 32 avec le bord périphérique 34 de l'implant. Ces chanfreins évitent la création de zones en saillie qui risqueraient de se coincer, dans certaines configurations d'utilisation de la prothèse P, contre les composants 1 ou 2.

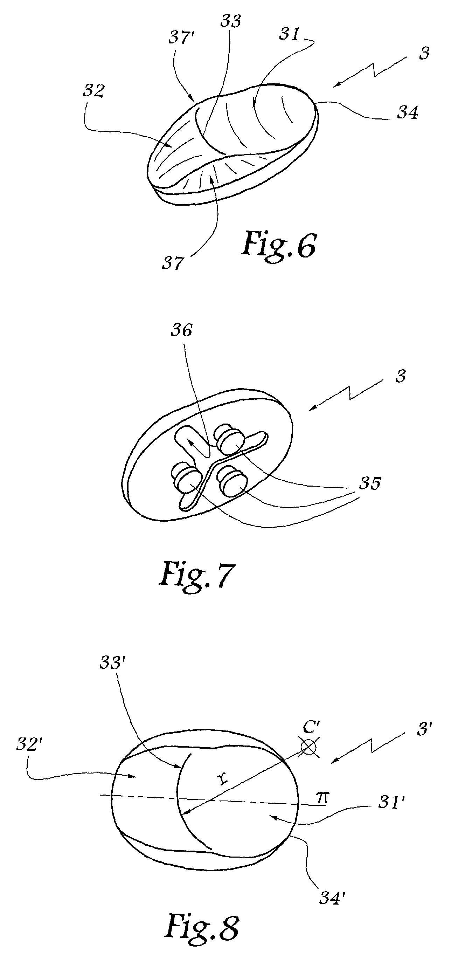

Dans le second mode de réalisation de l'invention représenté à la figure 8, l'implant 3' comprend également une surface articulaire externe 31', une surface articulaire interne 32' et une arête de jonction 33'.

Ce mode de réalisation diffère des précédents, en ce que le centre de courbure C' de l'arête 33' n'est pas situé dans le plan transversal médian π de l'implant, qui est le plan de coupe de la figure 2 pour l'implant 3, mais est décalé par rapport à ce plan π, de telle sorte que l'arête 33' n'est pas symétrique par rapport à ce plan π à la différence de l'arête 33 du premier mode de réalisation. Cette variante permet d'accentuer ou de diminuer l'effet de pivotement dans le sens de la flèche F₃. Bien entendu, le centre de courbure C' pourrait être défini de l'autre côté du plan π à la figure 8 et/ou au niveau du bord périphérique 34' de l'implant 3', le rayon de courbure r' de l'arête 33' étant adapté en conséquence.

## Revendications

1. Implant rotulien pour articulation prothétique totale ou partielle de genou, ledit implant incorporant une surface articulaire externe et une surface articulaire interne prévues pour coopérer respectivement avec une berge externe et une berge interne d'une trochlée fémorale ou d'un composant prothétique fémoral et reliées entre elles par une arête de transition, ladite surface articulaire externe étant concave dans un plan (figure 4) parallèle au plan sagittal (S) de l'articulation et dans un plan transversal (figure 2), **caractérisé en ce que** ladite arête (33, 33') est courbe en vue de face dudit implant (3, 3'), avec une concavité tournée vers ladite surface articulaire externe (31, 31').

2. Implant selon la revendication 1, **caractérisé en ce que** le centre de courbure (C, C') de ladite arête (33, 33') est situé à l'extérieur ou à la périphérie dudit implant (3, 3') en vue de face.

3. Implant selon l'une des revendications précédentes, **caractérisé en ce que** ladite surface articulaire externe (31, 31') est une surface de révolution.

4. Implant selon l'une des revendications précédentes, **caractérisé en ce qu'**il a, en vue de face, une forme globalement ovoïde.

5. Implant selon l'une des revendications précédentes, **caractérisé en ce que** ladite surface articulaire interne (32, 32') est plane ou convexe dans un plan (figure 5) parallèle au plan sagittal (S) de l'articulation.

6. Implant selon l'une des revendications précédentes, **caractérisé en ce qu'**il comprend deux chanfreins latéraux (37, 37') s'étendant selon des directions globalement perpendiculaires au plan sagittal (S) et reliant les bords respectifs (31a, 32a) desdites surfaces articulaires (31, 32) à un bord périphérique (34) dudit implant (3).

7. Implant selon l'une des revendications précédentes, **caractérisé en ce qu'**il est réalisé dans une pièce plastique monobloc.

8. Prothèse (P) totale ou partielle du genou, **caractérisée en ce qu'**elle comprend un implant (3, 3') selon l'une des revendications précédentes.
